# FASCICULE DE BREVET EUROPEEN

(11) **EP 0 556 118 B1**
(45) Date de publication et mention de la délivrance du brevet: **16.04.1997**
(21) Numéro de dépôt: 93400339.3
(22) Date de dépôt: 10.02.1993
(51) Int. Cl.: G21F 9/16

(54) **Procédé d'inclusion dans des résines (méth)acryliques de substances de consistance liquide à solide**
Verfahren zur Einbettung von flüssigen bis zu festen Substanzen in (meth)acrylischen Harzen
Process for including substances of liquid to solid consistency in (meth)acrylic resins

(30) Priorité: 13.02.1992 FR 9201638
(43) Date de publication de la demande: 18.08.1993
(73) Titulaire: ELF ATOCHEM S.A., 92800 Puteaux (FR)
(72) Inventeur: Legros, Robert, F-60940 Monceaux (FR); Wiegert, Bernard, F-60870 Brenouille (FR); Zeh, Jean-Luc, F-57350 Spicheren (FR)
(74) Mandataire: Chaillot, Geneviève

(56) Documents cités:
- EP-A- 0 315 462
- DE-A- 3 505 886
- GB-A- 2 093 854
- DATABASE WPIL Derwent Publications Ltd., London, GB; AN 84-091813 & JP-A-59 038 698
- DATABASE WPIL Derwent Publications Ltd., London, GB; AN 86-185538 &JP-A-61 117 496

## Description

La présente invention concerne l'inclusion, au sein de résines (méth)acryliques, de substances diverses ayant une consistance liquide à solide. Ce procédé d'inclusion permet d'obtenir de nouveaux matériaux composites, tels que des matériaux contenant de l'eau incluse sous forme de billes ou nodules, applicables entre autres dans le domaine des prothèses osseuses et dentaires. Le procédé d'inclusion permet aussi le conditionnement de déchets industriels divers, tels que les déchets mercuriels et les déchets contaminés comprenant des matériaux échangeurs de cations et d'anions, constitués, par exemple, par des résines cationiques ou par un mélange de résines cationiques et anioniques contaminées par des éléments radioactifs.

Ces résines sont notamment des résines de polystyrène réticulé avec du divinylbenzène, qui comportent des groupements sulfoniques SO₃H (résines cationiques) ou un mélange de telles résines avec des résines de polystyrène réticulé avec du divinylbenzène qui comportent des fonctions OH fixées sur un groupement ammonium quaternaire (résines anioniques).

Lorsque des résines échangeuses de cations sont utilisées pour purifier des eaux contaminées, notamment des effluents d'installations nucléaires, elles subissent au bout d'un certain temps des phénomènes de dégradation et de saturation et perdent en conséquence leur efficacité. Il s'agit alors de conditionner ces résines échangeuses d'ions usées qui ont fixé au cours de leur utilisation un certain nombre de radioéléments tout en assurant une bonne rétention de leur radioactivité.

Il est connu d'incorporer des résines échangeuses d'ions contaminées par des éléments radioactifs dans des résines thermodurcissables polymérisables à la température ambiante. Ces résines, qui sont constituées par exemple par un polyester insaturé ou une résine époxyde, sont polymérisées de façon à obtenir un bloc solide. Cependant, ce procédé qui est tout à fait satisfaisant pour les résines anioniques, ne peut être utilisé selon le même mode opératoire pour des résines cationiques qui ne sont pas totalement usées, c'est-à-dire qui comportent encore des ions H⁺ qui sont capables de consommer certains des réactifs utilisés pour le durcissement de la résine.

Pour résoudre ce problème, on a proposé de soumettre les résines échangeuses d'ions usées, non totalement saturées à un prétraitement au moyen d'une solution aqueuse d'un composé basique capable de bloquer les sites actifs des résines cationiques. Toutefois, la réalisation d'un tel prétraitement en phase liquide présente divers inconvénients. En effet, il nécessite une installation de prétraitement complémentaire en amont de l'installation de conditionnement et il entraîne de plus la production d'effluents contaminés résultant du relargage dans la phase liquide de prétraitement d'une partie des radioéléments qui étaient fixés sur les résines échangeuses d'ions.

Par ailleurs, il faut souligner que le procédé de conditionnement de déchets contaminés comprenant des résines échangeuses d'ions à l'aide de résines polyesters ou époxydes conduit à un matériau enrobé qui présente des caractéristiques mécaniques qui ne sont pas satisfaisantes. En effet, il est indispensable que le matériau fini possède de bonnes caractéristiques mécaniques lui permettant de pouvoir vieillir dans le temps sans trop subir de modifications mécaniques.

Par la demande de brevet allemand DE-A-0 315 462, on connaît un procédé de solidification ou d'encapsulation de compositions contenant une phase aqueuse sensiblement continue comprenant les opérations consistant à incorporer uniformément dans les compositions des particules de polymère acrylique coeur-écorce, puis à neutraliser les particules de polymère par incorporation dans les compositions d'une base organique ou minérale. Les particules de polymère neutralisées gonflent et absorbent sensiblement toute la phase aqueuse.

Le besoin se fait donc sentir de mettre au point un procédé d'inclusion qui permette d'éviter tous les inconvénients des procédés antérieurs. D'une manière générale, il est apparu intéressant de pouvoir disposer d'un procédé d'inclusion de déchets de tous types, solides et/ou liquides, entre autres, de systèmes aqueux ne contenant pas nécessairement de polluants. En effet, les bonnes caractéristiques mécaniques des blocs d'inclusion d'eau rendent ceux-ci intéressants comme nouveaux matériaux composites.

La présente invention a donc d'abord pour objet un procédé d'inclusion dans une résine d'au moins une substance de consistance liquide à solide, caractérisé en ce qu'on enrobe la (ou les) substance(s) à inclure dans une résine (méth)acrylique obtenue in situ par polymérisation directe, sans préparation préalable d'un sirop de prépolymère, à partir d'un système monomère à base d'au moins un monomère (méth)acrylique présentant un point d'ébullition compris entre 330 et 380°C (1,01 x 10⁵ Pa (760 mmHg)).

Conformément à un mode de réalisation particulièrement préféré, on utilise un système monomère à base d'au moins un monomère (méth)acrylique à point d'ébullition élevé, choisi parmi le dicyclopentadiényloxyéthyle méthacrylate (DCPOEMA) et son homologue époxydé (monomère de base), le cas échéant en présence d'au moins un monomère réticulant choisi notamment parmi les di- ou tri-(méth)acrylates, tels que le triméthylolpropane triméthacrylate, l'hexanediol diacrylate, le triméthylolpropaneoxyéthyle triacrylate, le glycéroltrioxypropyle triacrylate, l'éthylèneglycol diméthacrylate et le tripropylèneglycol diacrylate.

Le système monomère peut également contenir au moins un comonomère additionnel, choisi parmi le méthacrylate ou l'acrylate d'isobornyle, l'acide acrylique ou méthacrylique.

Selon l'invention, les monomères (méth)acryliques mis en oeuvre pour le procédé ont des points d'ébullition élevés. Pour le ou les monomère(s) de base, comme indiqué, il est compris entre 330 et 380°C (1,01 x 10⁵ Pa (760 mmHg)); pour le ou les monomères réticulants, le point d'ébullition varie entre 250 et 350°C, et pour le ou les monomères additionnels, autour de 270°C.

On peut également ajouter au système monomère au moins un agent épaississant choisi parmi les oligomères (méth)acryliques tels que le diméthacrylate de diglycidyle du bisphénol ; et le fumarate de diglycidyle du bisphénol.

Ainsi, afin d'épaissir fortement le milieu pour éviter une éventuelle décantation de déchets solides sous forme de fines particules, il a, entre autres, été possible de mélanger avec le DCPOEMA, jusqu'à 30% en poids d'oligomères acryliques ou méthacryliques tels que le diméthacrylate de diglycidyle du bisphénol ou jusqu'à 20% en poids de fumarate de diglycidyle du bisphénol.

De façon connue, on utilise un système monomère contenant au moins un initiateur de polymérisation choisi parmi un peroxyde : peroxyde de benzoyle, ou de lauryle, ou un percarbonate : percarbonate d'alcoyle par exemple, et au moins un activateur choisi de façon classique parmi les amines tertiaires : triméthylamine, diméthylparatoluidine, tétraméthylaniline, utilisées individuellement ou en mélange, ou parmi des activateurs organométalliques : de cobalt, de zirconium, ou de vanadium. Le ou les initiateurs sont généralement utilisés à raison de 0,5 à 5% en poids par rapport au poids de tous les autres constituants du mélange réactionnel. Quant aux activateurs, ils sont utilisés en quantité comprise entre 0,01 à 4% en poids par rapport au poids des autres constituants du mélange réactionnel. L'initiation radicalaire avec le système catalytique peroxyde de benzoyle - diméthylparatoluidine peut s'effectuer à basse température entre 5 et 30°C.

Il est aussi possible de mettre en oeuvre de façon connue des agents de transfert, tels que le dodécylmercaptan, ainsi que des agents émulsifiants et des agents tensioactifs.

Le procédé selon la présente invention consiste à ajouter dans un réacteur métallique par exemple, les monomères, la ou les substances à inclure de type liquide, boues ou broyats, sous agitation, puis un initiateur de polymérisation, un activateur et, éventuellement, un agent de transfert. La température du mélange agité s'élève au plus à 100°C au bout d'un temps égal au plus à 40 minutes. En fin de polymérisation, le produit fini est avantageusement cuit à une température comprise entre 50 et 85°C pendant 2 à 15 heures.

Dans le cas où l'on souhaite inclure des substances solides sous forme de blocs, on recouvre ces derniers par le mélange réactionnel de polymérisation, préparé comme ci-dessus. Suivant la température du milieu, le durcissement s'effectue en un laps de temps de l'ordre de 5-60 minutes.

Selon la présente invention, on peut par exemple préparer un bloc d'inclusion d'une substance choisie parmi :
- les résines échangeuses d'ions contaminées par des éléments radioactifs, comme décrit plus en détail ci-dessus ;
- de l'eau, laquelle peut le cas échéant, contenir des polluants, tels que des ions monovalents ou polyvalents, par exemple des sels de cobalt, de fer, de chrome, de bore, de césium, à des concentrations relativement élevées, pouvant aller jusqu'à 50% en poids, ou plus, même dans des conditions de pH extrêmes (pH 1) ;
- des déchets en phase solide, sous forme de blocs plus ou moins volumineux ou de broyats (de granulométrie pouvant par exemple être comprise entre quelques millimètres et quelques dizaines de millimètres), ou en phase liquide, tels que du béton mercuriel, des boues mercurielles, des bougies de graphite, lesdits déchets pouvant être inclus avec remplacement par de l'eau d'une partie de la teneur en monomère(s).

La présente invention concerne en outre :
- un bloc d'inclusion de résines échangeuses d'ions, obtenu par le procédé tel que défini ci-dessus, caractérisé en ce qu'il a été fabriqué en mettant en oeuvre les quantités suivantes des différents produits :
   - résines échangeuses d'ions : environ 30 à 75, de préférence, 50 à 65, parties en poids, ces résines contenant une quantité d'eau comprise en général entre 40 et 65% en poids ;
   - monomère(s) de base : environ 25 à 60, de préférence, 35 à 50, parties en poids ;
   - monomère(s) réticulant(s) : environ 0,5 à 10, de préférence, 1 à 5, parties en poids ;
   - monomère(s) additionnel(s) éventuel(s) : jusqu'à 10, de préférence, 4 à 8, parties en poids environ ;
- un bloc d'inclusion d'eau contenant le cas échéant au moins un élément polluant, obtenu par le procédé tel que défini ci-dessus, caractérisé par le fait qu'il contient notamment jusqu'à environ 40% en poids, par exemple de 10-40% en poids, d'eau, incluse sous forme de billes ou nodules d'un diamètre généralement inférieur à 150 µm ;
- un bloc d'inclusion de déchets de type béton mercuriel, boues mercurielles ou bougies de graphite, obtenu par le procédé tel que défini ci-dessus, le cas échéant avec remplacement par de l'eau d'une partie du ou des monomères, ledit bloc pouvant alors avoir, entre autres, la composition suivante :
   - déchets : 68 à 70 parties en poids environ ;
   - monomères : 23 à 22 parties en poids environ ;
   - eau : 9 à 8 parties en poids environ.

La présente invention concerne également l'utilisation des blocs d'inclusion tels que définis ci-dessus pour le stockage de polluants industriels, notamment de substances contaminées par des éléments radioactifs, ainsi que l'utilisation des blocs d'inclusion d'eau, tels que définis ci-dessus, comme matériaux de prothèse.

Les exemples suivants illustrent la présente invention : toutes les quantités sont exprimées en poids. Le dicyclopentadiényloxyéthyle méthacrylate est désigné par l'abréviation DCPOEMA.

### EXEMPLES 1 à 12 : Inclusion de résines échangeuses d'ions

### EXEMPLE 1

Dans un cylindre métallique qui est à température ambiante (20°C), on introduit 25 parties de résine échangeuse anionique présentant un extrait sec entre 35 et 40 %, et contaminée par des éléments radioactifs.

Sous agitation, on ajoute :
- 24,5 parties de DCPOEMA
- 1 partie de peroxyde de benzoyle à 50%
- 0,5 partie de triméthylolpropaneoxyéthyle triacrylate
- 0,025 partie de diméthylparatoluidine.

La température s'élève à 57°C en 15 minutes.

On effectue ensuite une cuisson isotherme de l'ensemble à 57°C pendant 3 heures.

Le bloc d'inclusion, parfaitement homogène, présente un point de ramollissement VICAT sous 1 kg de 45°C et une contrainte à la rupture de 55 da N/cm². Le ramollissement VICAT est déterminé selon la Norme AFNOR T51021, la contrainte à la rupture selon la Norme AFNOR T51101.

### EXEMPLE 2

L'exemple 1 est répété en utilisant les produits suivants :
- résine échangeuse anionique 25 parties
- DCPOEMA 19,5 parties
- peroxyde de benzoyle (50%) 2 parties
- triméthylolpropane triméthacrylate 2 parties
- octoate de cobalt à 6% 0,5 partie
- diméthylparatoluidine 1 partie

La température s'élève à 70°C en 1 minute.

Après cuisson thermique réalisée à 68-70°C environ pendant 3 heures, le bloc présente les caractéristiques suivantes :
- point de ramollissement VICAT sous 1 kg : 45°C
- contrainte à la rupture : 60 daN/cm²

### EXEMPLE 3

L'exemple 1 est répété en mettant en oeuvre les produits suivants :
- résine échangeuse anionique 25 parties
- DCPOEMA 17,5 parties
- tripropylèneglycol diacrylate 2 parties
- méthacrylate d'isobornyle 2 parties
- octoate de cobalt à 6% 0,5 partie
- peroxyde de benzoyle à 50% 2 parties
- diméthylparatoluidine 1 partie

La température s'élève à 77°C en 1 minute.

Après traitement thermique réalisé comme dans les exemples précédents, le bloc présente les caractéristiques suivantes :
- point de ramollissement VICAT sous 1 kg : 40°C
- contrainte à la rupture : 150 daN/cm²

### EXEMPLE 4

On opère comme dans l'exemple 1 en utilisant les produits suivants :
- résine échangeuse anionique 25 parties
- DCPOEMA 17,5 parties
- acide méthacrylique 2 parties
- méthacrylate d'isobornyle 2 parties
- octoate de cobalt à 6% 0,5 partie
- peroxyde de benzoyle à 50% 2 parties
- diméthylparatoluidine 1 partie

La température s'élève à 71°C en 1 minute.
- point de ramollissement VICAT sous 1 kg : 30°C
- contrainte à la rupture : 30 daN/cm²

### EXEMPLE 5

L'exemple 1 est répété en mettant en oeuvre une résine cationique :
- résine échangeuse cationique à 51% d'extrait sec 25 parties
- DCPOEMA 19,5 parties
- triméthylolpropane triméthacrylate 2 parties
- octoate de cobalt à 6% 0,5 partie
- peroxyde de benzoyle à 50% 2 parties
- diméthylparatoluidine 1 partie

La température du mélange s'élève à 72°C en 1 minute.

Après cuisson thermique, le bloc présente les caractéristiques suivantes :
- point de ramollissement VICAT sous 1 kg : 48°C
- contrainte à la rupture : 66 daN/cm²

### EXEMPLE 6

L'exemple 1 a été répété en utilisant la résine cationique de l'exemple 5.
- résine échangeuse cationique 25 parties
- DCPOEMA 17 parties
- tripropylèneglycol diacrylate 2 parties
- octoate de cobalt à 6% 0,5 partie
- peroxyde de benzoyle à 50% 2 parties
- diméthylparatoluidine 1 partie

La température du mélange s'élève à 60°C en 1 minute.

Après traitement thermique le bloc obtenu présente les caractéristiques suivantes :
- point de ramollissement VICAT sous 1 kg : 43°C
- contrainte à la rupture : 265 daN/cm²

### EXEMPLE 7

L'exemple 6 est répété en mettant en oeuvre les ingrédients suivants :
- résine échangeuse cationique 25 parties
- DCPOEMA 19,5 parties
- triméthylolpropane triacrylate 2 parties
- octoate de cobalt à 6% 0,5 partie
- peroxyde de benzoyle à 50% 2 parties
- diméthylparatoluidine 1 partie

La température du mélange s'élève à 76°C en 1 minute.

Après traitement thermique comme dans l'exemple 1 le bloc obtenu présente les caractéristïques suivantes :
- point de ramollissement VICAT sous 1 kg : 60°C
- rupture à la compression : 180 daN/cm²

### EXEMPLE 8

L'exemple 1 est répété :
- résine échangeuse cationique 25 parties
- DCPOEMA 17,5 parties
- tripropylèneglycol diacrylate 2 parties
- méthacrylate d'isobornyle 2 parties
- octoate de cobalt à 6% 0,5 partie
- peroxyde de benzoyle à 50% 2 parties
- diméthylparatoluidine 1 partie

La température du mélange s'élève à 68°C en 1 minute.
- point de ramollissement VICAT sous 1 kg : 50°C
- rupture à la compression : 70 daN/cm²

### EXEMPLE 9

L'exemple 1 est répété en mettant en oeuvre un mélange de résines échangeuses d'ions contaminées :
- résine échangeuse anionique 15 parties
- résine échangeuse cationique 10 parties
- DCPOEMA 17,5 parties
- tripropylèneglycol diacrylate 2 parties
- méthacrylate d'isobornyle 2 parties
- octoate de cobalt à 6% 0,5 partie
- peroxyde de benzoyle à 50% 2 parties
- diméthylparatoluidine 1 partie

La température du mélange s'élève à 76°C en 1 minute.

Après traitement thermique réalisé comme dans l'exemple 1, le bloc obtenu présente les caractéristiques suivantes :
- point de ramollissement VICAT sous 1 kg : 40°C
- rupture à la compression : 60 daN/cm²

### EXEMPLE 10

L'exemple 1 est répété en mettant en oeuvre les ingrédients suivants :
- résine échangeuse cationique 25 parties
- DCPOEMA 17 parties
- acrylate d'isobornyle 3 parties
- tripropylèneglycol diacrylate 1 partie
- triméthylolpropane triacrylate 1 partie
- peroxyde de benzoyle à 50% 1 partie
- diméthylparatoluidine 0,5 partie

La température du mélange s'élève à 72°C en 1 minute.
- point de ramollissement VICAT sous 1 kg : 52°C
- rupture à la compression : 190 daN/cm²

### EXEMPLE 11

L'exemple 1 est répété à l'aide des produits suivants :
- résine échangeuse cationique 25 parties
- DCPOEMA 17 parties
- acrylate d'isobornyle 3 parties
- tripropylèneglycol diacrylate 1 partie
- triméthylolpropane triacrylate 1 partie
- peroxyde de benzoyle à 50% 2 parties
- diméthylparatoluidine 0,25 partie

La température du mélange s'élève à 75°C en 2 minutes.
- point de ramollissement VICAT sous 1 kg : 55°C
- rupture à la compression : 175 daN/cm²

### EXEMPLE 12

L'exemple 1 est répété en mettant en oeuvre :
- résine échangeuse cationique 25 parties
- DCPOEMA époxydé 17 parties
- acrylate d'isobornyle 3 parties
- tripropylèneglycol diacrylate 1 partie
- triméthylolpropane triacrylate 1 partie
- peroxyde de benzoyle à 50% 2 parties
- diméthylparatoluidine 1,0 partie

La température du mélange s'élève à 80°C en 3 minutes.
- point de ramollissement VICAT sous 1 kg : 45°C
- rupture à la compression : 145 daN/cm²

### EXEMPLES 13 à 16 : Obtentions de blocs d'inclusion d'eau

Pour chaque exemple, on a préparé deux blocs d'inclusion respectivement à 30 et 40% en poids d'eau.

### Mode opératoire général

Dans un récipient en polyéthylène ou en fer, on a introduit l'eau, le mélange monomère contenant le DCPOEMA, les réticulants éventuels (tripropylèneglycol diacrylate, triméthylolpropane triacrylate ou triméthylol propane triméthacrylate), le comonomère éventuel (acrylate d'isobornyle), et le peroxyde de benzoyle. On a agité pendant 15 minutes de façon à créer une émulsion suffisamment stable pour la polymérisation, tandis que la température était amenée à 20°C ± 1°C. On a alors ajouté la diméthylparatoluidine, agité pendant 2 minutes, puis arrêté l'agitation. On a suivi le déroulement de la polymérisation par la mesure de la température du milieu réactionnel en fonction du temps : après une période d'inhibition de la polymérisation de quelques minutes pendant laquelle la température reste constante, la température augmente rapidement pour atteindre une valeur maximale inférieure à 100°C. Puis, elle diminue lentement jusqu'à la température ambiante.

On obtient alors un bloc homogène, coloré en beige rosé, ne présentant aucun suintement d'eau. Pour éviter toute présence de monomères résiduels, on a conduit dans chaque cas un recuit de 15 heures à 70°C.

Les différentes compositions des mélanges réactionnels mis en jeu sont les suivantes :

**Tableau 1**

| Composition du mélange réactionnel * | 13 | 14 | 15 | 16 |
|---|---|---|---|---|
| DCPOEMA | 340 | 340 | 440 | 340 |
| Acrylate d'isobornyle | 60 | 60 | | 60 |
| Tripropylèneglycol diacrylate | 20 | | | |
| Triméthylolpropane triacrylate | 20 | | | |
| Triméthylolpropane triméthacrylate | | 40 | | |
| Peroxyde de benzoyle | 5 | 5 | 5 | 5 |
| Diméthylparatoluidine | 1 | 1 | 1 | 1 |

| | | | | |
|---|---|---|---|---|
| * en parties en poids | | | | |

L'examen de la morphologie des matériaux par microscopie électronique à balayage montre que ces matériaux sont remplis de trous dont la taille est de 150 µm au maximum, la population de trous augmentant avec le pourcentage d'eau. L'eau est donc bien enrobée dans la matrice polymère, en étant présente dans celle-ci sous la forme de petites billes.

Par ailleurs, la dureté du matériau (point VICAT) augmente avec le pourcentage d'eau, de même que la contrainte maximale à l'écrasement :

| | contrainte maximale |
|---|---|
| 30% d'eau | 18,1 MPa |
| 40% d'eau | 23,4 MPa, |

ce qui laisse à penser que les billes d'eau se comportent comme une charge apportant une résistance au choc et à la compression du matériau.

La bonne compatibilité du DCPOEMA avec l'eau, son innocuité vis-à-vis de l'être humain, et son absence de perte d'eau, rend ces composites utiles dans le domaine des prothèses.

### EXEMPLES 17 à 20 : Inclusion de déchets divers

### EXEMPLE 17 : Inclusion de boues mercurielles diverses pouvant contenir jusqu'à 75% d'eau

On prépare la composition suivante :
- DCPOEMA 340 parties
- acrylate d'isobornyle 60 parties
- triméthylolpropane triméthacrylate 40 parties.

Dans 500 g de ce mélange monomère, on a introduit sous agitation 500 g de boues. On a laissé émulsionner sous agitation entre 10 et 30 minutes.

Puis, on a introduit 0,5 à 1% de peroxyde de benzoyle par rapport au mélange monomère, on a agité pendant 5 à 15 minutes, puis ajouté 0,1 à 0,4% de diméthylparatoluidine par rapport au mélange de monomères.

On a arrêté l'agitation, selon les systèmes catalytiques, au bout de 2 à 6 minutes.

### EXEMPLE 18 : Inclusion de divers bétons mercuriels broyés ayant un taux d'humidité variable

On a procédé de la même manière qu'à l'Exemple 17 en remplaçant les boues par différents bétons broyés chargés à raison de 50-60%.

### EXEMPLE 19 : Inclusion de divers blocs de bétons mercuriels

Dans un premier récipient, on a introduit les blocs de béton. Dans un second récipient, on a introduit le DCPOEMA ou un mélange de DCPOEMA et de comonomères tels que l'acrylate d'isobornyle, avec ou sans monomères réticulants, on a agité, puis on ajouté de 0,5 à 1% de peroxyde de benzoyle par rapport au mélange des monomères, agité encore pendant 10 minutes, puis ajouté 0,1 à 0,4% de diméthylparatoluidine par rapport au mélange des monomères. Le rapport béton/ mélange de monomères est compris entre 60/40 et 70/30.

Le mélange réactionnel est alors versé dans le premier récipient, pour recouvrir les blocs de béton. Suivant la température du milieu, le durcissement s'effectue entre 5 à 60 minutes.

### EXEMPLE 20 : Inclusion de blocs ou broyats de bougies de graphite ayant un taux d'humidité variable

On reproduit les modes opératoires des exemples 18 et 19 pour obtenir des blocs à inclusion de 50 à 70% de bougies de graphite.

## Revendications

1. Procédé d'inclusion dans une résine d'au moins une substance de consistance liquide à solide, caractérisé en ce qu'on enrobe la (ou les) substance(s) à inclure dans une résine (méth)acrylique obtenue in situ par polymérisation directe, sans préparation préalable d'un sirop de prépolymère, à partir d'un système monomère à base d'au moins un monomère (méth)acrylique présentant un point d'ébullition compris entre 330 et 380°C (1,01 x 10⁵ Pa (760 mmHg)).

2. Procédé selon la revendication 1, caractérisé en ce qu'on utilise un système monomère à base d'au moins un monomère (méth)acrylique à point d'ébullition élevé, choisi parmi le dicyclopentadiényloxyéthyle méthacrylate et son homologue époxydé (monomère de base), le cas échéant en présence d'au moins un monomère réticulant choisi notamment parmi les di- ou tri-(méth)acrylates tels que le triméthylolpropane triméthacrylate, l'hexanediol diacrylate, le triméthylolpropaneoxyéthyle triacrylate, le glycéroltrioxypropyle triacrylate, l'éthylèneglycol diméthacrylate et le tripropylèneglycol diacrylate.

3. Procédé selon l'une des revendications 1 et 2, caractérisé en ce qu'on utilise un système monomère contenant additionnellement au moins un comonomère choisi parmi le méthacrylate ou l'acrylate d'isobornyle, l'acide acrylique ou méthacrylique.

4. Procédé selon l'une des revendications 1 à 3, caractérisé en ce qu'on ajoute au système monomère au moins un agent épaississant choisi parmi les oligomères (méth)acryliques tels que le diméthacrylate de diglycidyle du bisphénol ; et le fumarate de diglycidyle du bisphénol.

5. Procédé selon l'une des revendications 1 à 4, caractérisé en ce qu'on utilise un système monomère contenant au moins un initiateur de polymérisation choisi parmi les peroxydes et les percarbonates, et au moins un activateur choisi parmi les amines tertiaires et les activateurs organométalliques.

6. Procédé selon l'une des revendications 1 à 5, caractérisé en ce qu'on soumet le bloc d'inclusion résultant de la polymérisation in situ à un recuit à une température comprise entre 50 et 85°C, pendant une durée de 2 à 15 heures.

7. Procédé d'inclusion selon l'une des revendications 1 à 6, caractérisé en ce qu'on prépare un bloc d'inclusion d'une substance choisie parmi :
- les résines échangeuses d'ions contaminées par des éléments radioactifs ;
- de l'eau, laquelle peut, le cas échéant, contenir des polluants, tels que des ions monovalents ou polyvalents, tels que des sels de cobalt, de fer, de chrome, de bore, de césium ;
- des déchets en phase solide, sous forme de blocs ou de broyats, ou en phase liquide, tels que du béton mercuriel, des boues mercurielles, des bougies de graphite, lesdits déchets pouvant être inclus avec remplacement par de l'eau d'une partie de la teneur en monomère(s).

8. Bloc d'inclusion de résines échangeuses d'ions, obtenu par le procédé tel que défini à l'une des revendications 1 à 6, caractérisé en ce qu'il a été fabriqué en mettant en oeuvre les quantités suivantes des différents produits :
- résines échangeuses d'ions : 30 à 75, de préférence, 50 à 65, parties en poids, ces résines contenant une quantité d'eau comprise en général entre 40 et 65% en poids ;
- monomère(s) de base : 25 à 60, de préférence, 35 à 50, parties en poids ;
- monomère(s) réticulant(s) : 0,5 à 10, de préférence, 1 à 5, parties en poids ;
- monomère(s) additionnel(s) éventuel(s) : 0 à 10, de préférence, 4 à 8, parties en poids.

9. Bloc d'inclusion d'eau contenant le cas échéant au moins un élément polluant, obtenu par le procédé tel que défini à l'une des revendications 1 à 6, caractérisé par le fait qu'il contient jusqu'à 40% en poids d'eau incluse sous forme de billes ou nodules d'un diamètre généralement inférieur à 150 µm.

10. Bloc d'inclusion de déchets de type béton mercuriel, boues mercurielles ou bougies de graphite, obtenu par le procédé tel que défini à l'une des revendications 1 à 6, le cas échéant avec remplacement d'une partie du ou des monomères par de l'eau, pouvant alors avoir, entre autres, la composition suivante :
- déchets : 68 à 70 parties en poids ;
- monomères : 23 à 22 parties en poids ;
- eau : 9 à 8 parties en poids.

11. Utilisation des blocs d'inclusion tels que définis à l'une des revendications 8 à 10 pour le stockage de polluants industriels, notamment de substances contaminées par des éléments radioactifs.

12. Utilisation des blocs d'inclusion d'eau, tels que définis à la revendication 9, comme matériaux de prothèse.

## Patentansprüche

1. Verfahren zur Einlagerung mindestens einer Substanz mit flüssiger bis fester Konsistenz in einem Harz, dadurch gekennzeichnet, daß man die einzulagernde(n) Substanz(en) in einem (Meth-)Acrylharz einlagert, das man in situ durch direkte Polymerisation, d.h. ohne vorgeschaltete Herstellung eines prepolymeren Sirups, ausgehend von einem Monomersystem auf Basis mindestens eines (Meth-)Acrylmonomers erhält, das einen Siedepunkt zwischen 330 und 380 °C (1,01 x 10⁵ Pa (760 mmHg)) aufweist.

2. Verfahren nach Anspruch 1, dadurch gekennzeichnet, daß man ein Monomersystem auf Basis mindestens eines (Meth-)Acrylmonomers mit einem hohen Siedepunkt verwendet, das ausgewählt ist aus Dicyclopentadienyloxyethylmethacrylat und seinem epoxydierten Homologen (Basismonomer), gegebenenfalls in Gegenwart mindestens eines vernetzenden Monomers, das insbesondere ausgewählt ist aus Di- oder Tri(meth-)acrylaten wie Trimethylolpropantrimethacrylat, Hexandioldiacrylat, Trimethylolpropanoxyethyltriacrylat, Glyceroltrioxypropyltriacrylat, Ethylenglykoldimethacrylat und Tripropylenglycoldiacrylat.

3. Verfahren nach Anspruch 1 oder 2, dadurch gekennzeichnet, daß man ein Monomersystem verwendet, das zusätzlich mindestens ein Comonomer enthält, das ausgewählt ist aus Isobornylmethacrylat oder -acrylat und Acryl- oder Methacrylsäure.

4. Verfahren nach einem der Ansprüche 1 bis 3 dadurch gekennzeichnet, daß man zu dem Monomersystem mindestens ein Verdickungsmittel hinzugibt, das ausgewählt ist aus (Meth-)Acryloligomeren wie Bisphenoldiglycidyldimethacrylat und Bisphenoldiglycidylfumarat.

5. Verfahren nach einem der Ansprüche 1 bis 4, dadurch gekennzeichnet, daß man ein Monomersystem verwendet, enthaltend mindestens einen Polymerisationsstarter, ausgewählt aus Peroxyden und Percarbonaten, und mindestens ein Aktivierungsreagenz, ausgewählt aus tertiären Aminen und Organometallaktivatoren.

6. Verfahren nach einem der Ansprüche 1 bis 5, dadurch gekennzeichnet, daß man den aus der In-situ-Polymerisation stammenden Einlagerungsblock einer Temperung bei einer Temperatur zwischen 50 und 85 °C während einer Dauer von 2 bis 15 Stunden aussetzt.

7. Verfahren zur Einlagerung nach einem der Ansprüche 1 bis 6, dadurch gekennzeichnet, daß man einen Einlagerungsblock einer Substanz herstellt, die ausgewählt ist aus:
- Ionenaustauscherharzen, die mit radioaktiven Elementen kontaminiert sind;
- Wasser, das gegebenenfalls Verschmutzungen enthalten kann wie ein- oder mehrwertige Ionen wie Kobalt-, Eisen-, Chrom-, Bor- und Cäsiumsalze;
- Abfällen in fester Phase in Form von Klumpen oder zerkleinerten Stücken oder in flüssiger Phase wie z.B. quecksilberhaltiger Beton, quecksilberhaltige Schlämme und Graphitkerzen, wobei diese Abfälle unter Ersetzen eines Teils des Monomerengehalts durch Wasser eingebettet werden können.

8. Einlagerungsblock mit eingelagerten Ionenaustauscherharzen, erhalten durch das Verfahren gemäß einem der Ansprüche 1 bis 6, dadurch gekennzeichnet, daß er unter Verwendung der folgenden Mengen der unterschiedlichen Produkte hergestellt wurde:
- Ionenaustauscherharze: 30 bis 75 Gewichtsteile, vorzugsweise 50 bis 65 Gewichtsteile, wobei diese Harze eine Wassermenge von im allgemeinen zwischen 40 und 65 Gew.-% enthalten;
- ein oder mehrere Basismonomere: 20 bis 60 Gewichtsteile, vorzugsweise 35 bis 50 Gewichtsteile;
- ein oder mehrere vernetzende Monomere: 0,5 bis 10 Gewichtsteile, vorzugsweise 1 bis 5 Gewichtsteile;
- gegebenenfalls ein oder mehrere zusätzliche Monomere: 0 bis 10 Gewichtsteile, vorzugsweise 4 bis 8 Gewichtsteile.

9. Einlagerungsblock mit eingelagertem Wasser, enthaltend gegebenenfalls mindestens ein verschmutzendes Element und erhalten durch das Verfahren nach einem der Ansprüche 1 bis 6, dadurch gekennzeichnet, daß er bis zu 40 Gew.-% eingeschlossenes Wasser in Form von Kügelchen oder Knöllchen mit einem Durchmesser von im allgemeinen weniger als 150 um enthält.

10. Einlagerungsblock mit eingelagerten Abfällen vom Typ quecksilberhaltiger Beton, quecksilberhaltige Schlämme und Graphitkerzen, erhalten durch das Verfahren nach einem der Ansprüche 1 bis 6, gegebenenfalls unter Ersetzen eines Teils des oder der Monomeren durch Wasser, wobei er dann unter anderem die folgende Zusammensetzung aufweisen kann:
- Abfälle: 68 bis 70 Gewichtsteile;
- Monomere: 23 bis 22 Gewichtsteile;
- Wasser: 9 bis 8 Gewichtsteile.

11. Verwendung von Einlagerungsblöcken nach einem der Ansprüche 8 bis 10 zur Lagerung von industriellen Abfällen, insbesondere von Substanzen, die mit radioaktiven Elementen kontaminiert sind.

12. Verwendung von Einlagerungsblöcken mit eingelagertem Wasser gemäß Anspruch 9 als Materialien für Prothesen.

## Claims

1. Process for inclusion in a resin of at least one substance of liquid to solid consistency, characterised in that the substance (s) to be included is/are embedded in a (meth)acylic resin obtained in situ by direct polymerisation, without prior preparation of a prepolymer syrup, using a monomer system based on at least one (meth)acylic monomer possessing a boiling point between 330 and 380°C (1.01 x 10⁵ Pa (760 mm Hg)).

2. Process according to Claim 1, characterised in that a monomer system is used based on at least one (meth)acrylic monomer having a high boiling point, chosen from dicyclopentadienyloxyethyl methacrylate and its epoxidised homologue (base monomer), where appropriate in the presence of at least one crosslinking monomer chosen, in particular, from di- and tri(meth)acrylates, such as trimethylolpropane trimethacrylate, hexanediol diacrylate, trimethylolpropaneoxyethyl triacrylate, glyceroltrioxypropyl triacrylate, ethylene glycol dimethacrylate and tripropylene glycol diacrylate.

3. Process according to one of Claims 1 and 2, characterised in that a monomer system is used additionally containing at least one comonomer chosen from isobornyl methacrylate or acrylate and acrylic or methacrylic acid.

4. Process according to one of Claims 1 to 3, characterised in that at least one thickening agent chosen from (meth)acrylic oligomers such as bisphenol diglycidyl dimethacrylate and bisphenol diglycidyl fumarate is added to the monomer system.

5. Process according to one of Claims 1 to 4, characterised in that a monomer system is used containing at least one polymerisation initiator chosen from peroxides and percarbonates, and at least one activator chosen from tertiary amines and organometallic activators.

6. Process according to one of Claims 1 to 5, characterised in that the inclusion block resulting from the in situ polymerisation is subjected to a postcuring at a temperature of between 50 and 85°C for a period of 2 to 15 hours.

7. Inclusion process according to one of Claims 1 to 6, characterised in that an inclusion block of a substance chosen from the following is prepared:
- ion exchange resins contaminated with radioactive elements;
- water, which can, where appropriate, contain pollutants, such as monovalent or polyvalent ions, such as cobalt, iron, chromium, boron or caesium salts;
- solid-phase wastes, in the form of blocks or pulverised preparations, or liquid-phase wastes such as mercurial concrete, mercurial sludges or graphite rods, it being possible for the said wastes to be included with replacement of a portion of the monomer content by water.

8. Inclusion block of ion exchange resins, obtained by the process as defined in one of Claims 1 to 6, characterised in that it has been manufactured employing the following amounts of the different products:
- ion exchange resins: 30 to 75, and preferably 50 to 65, parts by weight, these resins containing an amount of water generally between 40 and 65 % by weight;
- base monomer(s): 25 to 60, and preferably 35 to 50, parts by weight;
- crosslinking monomer(s): 0.5 to 10, and preferably 1 to 5, parts by weight;
- possible additional monomer(s): 0 to 10, and preferably 4 to 8, parts by weight.

9. Water-inclusion block containing, where appropriate, at least one pollutant, obtained by the process as defined in one of Claims 1 to 6, characterised in that it contains up to 40 % by weight of water included in the form of globules or nodules generally less than 150 µm in diameter.

10. Inclusion block of wastes of the mercurial concrete, mercurial sludge or graphite rod type, obtained by the process as defined in one of Claims 1 to 6, where appropriate with replacement of a portion of the monomer or monomers by water, it then being able to have, inter alia the following composition:
- wastes : 68 to 70 parts by weight;
- monomers : 23 to 22 parts by weight;
- water : 9 to 8 parts by weight.

11. Use of the inclusion blocks as defined in one of Claims 8 to 10, for the storage of industrial pollutants, in particular substances contaminated with radioactive elements.

12. Use of the water-inclusion blocks as defined in Claim 9, as prosthesis materials.
